(19) **Europäisches Patentamt**

**European Patent Office**

**Office européen des brevets**

(11) Veröffentlichungsnummer: **0 220 135 B1**

(12) ## EUROPÄISCHE PATENTSCHRIFT

(45) Veröffentlichungstag der Patentschrift: **21.11.91**

(51) Int. Cl.⁵: **C07C 50/24**, C07C 46/00, C07C 46/06

(21) Anmeldenummer: **86810459.7**

(22) Anmeldetag: **17.10.86**

(54) **Verfahren zur Herstellung von Chloranil.**

(30) Priorität: **23.10.85 CH 4553/85**

(43) Veröffentlichungstag der Anmeldung:
**29.04.87 Patentblatt 87/18**

(45) Bekanntmachung des Hinweises auf die
Patenterteilung:
**21.11.91 Patentblatt 91/47**

(84) Benannte Vertragsstaaten:
**CH DE FR GB IT LI**

(56) Entgegenhaltungen:
**DE-A- 2 632 565**
**DE-C- 511 209**
**GB-A- 274 700**

(73) Patentinhaber: **CIBA-GEIGY AG**
**Klybeckstrasse 141**
**CH-4002 Basel(CH)**

(72) Erfinder: **Albrecht, Bernhard**
**Im Laig 173**
**CH-4463 Buus(CH)**
Erfinder: **Beyrich, Jürgen, Dr.**
**Am Tischliweg 3**
**WE-7859 Huttingen(DE)**
Erfinder: **Schaulin, Rudolf, Dr.**
**Scäferstrasse 62**
**CH-4125 Riehen(CH)**

**Beschreibung**

Die Erfindung betrifft ein neues Verfahren zur Herstellung von Chloranil, ausgehend von Chinon, Hydrochinon oder deren Chlorderivaten, bei dem Salzsäure mit elementarem Chlor unter Druck umgesetzt wird.

Chloranil ist ein wichtiges Zwischenprodukt für Textilfarbstoffe und dient z.B. zur Herstellung von Farbstoffen der Dioxazin-Reihe. In der Literatur sind zahlreiche Verfahren zur Herstellung dieser Verbindung beschrieben. Als Ausgangsmaterial dienen Phenol, Hydrochinon, Anilin, Cyclohexan, Benzol oder z.B. auch Pentachlorphenol. Umgesetzt werden diese Verbindungen allgemein mit elementarem Chlor in Mineralsäuren, gegebenenfalls in Gegenwart von Sauerstoff. Anstelle von Chlor kann auch Wasserstoffperoxid/Salzsäure verwendet werden.

H. Lübbecke und P. Boldt geben ein Verfahren an, wonach Hydrochinon mit Wasserstoffperoxid/Salzsäure unter Zusatz von Magnesiumchlorid in einer Ausbeute von 88 % zu Chloranil umgesetzt wird (Angew. Chem. 88 (1976) 641). Vorteil dieses Verfahrens ist, dass keine spezielle Chlorierungsanlage benötigt wird, nachteilig ist, dass relativ verdünnt gearbeitet werden muss, was sich ungünstig auf die Volumenausbeute auswirkt. Vorteil der oxidativen Chlorierung von Cyclohexan bzw. Benzol, einer weiteren Herstellungsvariante, ist zweifellos die Verwendung von Grundchemikalien als Ausgangsmaterial. Als Chlorierungsmittel verwendet man Chlor bzw. Chlorwasserstoff in Gegenwart von Sauerstoff unter Zusatz eines Katalysators (siehe GB-B-1,048,799). Der schwerwiegende Nachteil dieses Verfahrens ist jedoch, dass toxische Nebenprodukte entstehen können, wie beispielsweise das Octachlorobenzo-p-dioxin (L.P. Esposito et al. EPA-600/2-80-197(1980) Seite 73).

Eine seit langem bekannte Methode zur Herstellung von Chloranil besteht darin, Chinon bzw. Hydrochinon in konzentrierter Salzsäure zu lösen und in die heisse Lösung während mehreren Stunden Chlorgas einzuleiten. Man erhält auf diese Weise nahezu quantitativ das gewünschte Produkt (R. Schuloff, R. Pollak Chemiker-Zeitung 58 (1932), Seite 570). Dieses Verfahren ist jedoch, wegen des hohen Chlorverbrauchs im technischen Massstab nicht durchführbar und auch bei Rückführung des Chlors wegen der hierzu erforderlichen Zusatzapparaturen unwirtschaftlich. Hinzu kommt, dass während der Reaktion erhebliche Mengen an Chloranil aus dem Reaktor sublimieren und sich im Kühler niederschlagen.

Arbeitet man in einem geschlossenen Reaktor unter Druck, so zeigt sich, dass man praktisch mit der stöchiometrischen Menge an Chlor auskommt und in gleich guter Ausbeute ein qualitativ einwandfreies Produkt erhält. Dieses Verfahren benötigt zwar einen Autoklaven als Reaktor, ist ansonsten aber denkbar einfach in der Durchführung. Dennoch wird es nicht restlos den hohen Anforderungen, die an grosstechnische Verfahren gestellt werden, gerecht, handelt es sich doch hier um eine exotherme Reaktion, die in einem geschlossenen System durchgeführt wird, was aus sicherheitstechnischen Gründen nicht ganz unbedenklich ist.

Gefunden wurde nun, dass sich Chloranil auf einfache und sicherheitstechnische unbedenkliche Weise in hoher Ausbeute herstellen lässt, wenn man anstatt den Reaktor mit Hydrochinon und Chlor zu laden und unter Druck aufzuheizen, Hydrochinon, Chinon oder deren Chlorderivate und Chlor getrennt voneinander simultan unter Druck in einen Reaktor dosiert, in dem Salzsäure als Reaktionsmedium vorgelegt wurde. Die Zuführung an Hydrochinon, Chinon oder deren Chlorderivaten und Chlor kann dann jederzeit unterbrochen werden, so dass es zu keiner Akkumulation an nicht umgesetztem Hydrochinon, Chinon oder deren Chlorderivat oder einem gefährlichen Druckanstieg kommt.

Gegenstand der vorliegenden Erfindung ist somit ein Verfahren zur Herstellung von Chloranil durch Chlorieren von Hydrochinon, Chinon oder deren Chlorderivate in Salzsäure, das dadurch gekennzeichnet ist, dass man die Salzsäure vorlegt und unter Druck Hydrochinon, Chinon oder deren Chlorderivate und Chlor in getrennten Strömen gleichzeitig zudosiert.

Das als Ausgangsmaterial verwendete Hydrochinon, Chinon oder deren Chlorderivate sind wohlbekannte Verbindungen; es handelt sich dabei beim Chinon um p-Chinon sowie bei den Chlorderivaten um Mono-, Di- oder Trichlor-p-chinon, sowie Mono-, Di- oder Trichlorhydrochinon. Eingesetzt wird dieses Ausgangsmaterial zweckmässigerweise in Form einer wässrigen Anschlämmung, Suspension oder Lösung; üblicherweise wird 1 Mol Ausgangsmaterial in 0,2 bis 1 Liter Wasser angeschlämmt. Es ist natürlich auch möglich, das Ausgangsmaterial in Salzsäure anzuschlämmen oder zu lösen. Andererseits sorgt der bei der Reaktion entstehende Chlorwasserstoff dafür, dass es auch bei Verwendung einer wässrigen Ausgangsmaterialanschlämmung praktisch zu keiner Verdünnung der Salzsäure im Reaktor kommt. Die wässrige oder salzsaure Suspension bzw. Lösung wird mittels einer geeigneten Vorrichtung unter Druck in den Reaktor gefördert.

Bei der als Reaktionsmedium verwendeten Salzsäure handelt es sich zweckmässigerweise um konzentrierte Salzsäure und zwar in erster Linie um Salzsäure einer Konzentration von 30 bis 37 %. Dabei werden für 1 Mol Ausgangsmaterial im allgemeinen 0,5 bis 1 kg konzentrierte Salzsäure vorgelegt.

Durchgeführt wird die Reaktion mit elementarem Chlor (Chlorgas oder flüssiges Chlor) als Chlorierungsmittel, wobei es sich als vorteilhaft erweist, z.B. bei Chinon oder Hydrochinon und Chlor dieses in einem solchen Verhältnis in den Reaktor einzuleiten, dass auf 1 Mol Chinon oder Hydrochinon 4 bis 6 Mol Chlor kommen. Geringere Mengen an Chlor führen zu einem unvollständigen Umsatz des Chinons oder Hydrochinons, während ein grösserer Ueberschuss an Chlor im allgemeinen unwirtschaftlich ist, und keine Ausbeutesteigerung oder Qualitätsverbesserung ergibt. Allgemein geht man so vor, dass man zu Beginn der Reaktion, noch bevor man mit der Dosierung des Chinons oder Hydrochinons beginnt, Chlorgas in den Reaktor einleitet, bis die Salzsäure mit Chlor gesättigt und ein geringer Ueberdruck von einigen Bar bis 10 bar erreicht ist. Dann beginnt man mit der Dosierung der Chinon- bzw. Hydrochinonsuspension und leitet weiter Chlorgas ein. Dabei steigt der Druck gegebenenfalls noch etwas an und die Zufuhr an Hydrochinon und Chlor wird so geregelt, dass sich ein Druck von ca. 10 bar einstellt.

Durchgeführt wird die Reaktion vorteilhaft bei einer Temperatur von 60 bis 150°C, vorzugsweise zwischen 80 und 150°C, vor allem zwischen 80 und 120°C und insbesondere bei einer Temperatur von ca. 120°C. Reaktionstemperaturen, die deutlich über diesem vorteilhaften Temperaturbereich liegen, führen zu einem starken Druckanstieg und es besteht die Gefahr einer vermehrten Bildung an Nebenprodukten.

In Abhängigkeit von der Reaktionstemperatur und der Dosiergeschwindigkeit des Chlorgases stellt sich, wie bereits erwähnt, ein Ueberdruck ein, und zwar arbeitet man vorteilhaft bei einem Druck von 3 bis 40 bar, vor allem 5 bis 40 bar, insbesondere bei einem Druck von ca. 8 bis 15 bar bzw. 3 bis 12 bar.

Zur Aufarbeitung kann man das Reaktionsgemisch zunächst mit einem Inertgas, z.B. Stickstoff durchspülen und auf diese Weise überschüssiges Chlor entfernen. Letzte Chlorreste können gegebenenfalls noch mittels Hydrogensulfit zerstört werden. Anschliessend isoliert man das im Reaktionsmedium unlösliche Produkt mit Hilfe üblicher Trennmethoden, wie z.B. durch Filtrieren, Dekantieren oder Zentrifugieren. Vorteilhaft filtriert man das Produkt ab, wäscht es gegebenenfalls neutral und trocknet es anschliessend. Die als Filtrat zurückbleibende Salzsäure kann direkt im nachfolgenden Ansatz wieder vorgelegt und erneut als Reaktionsmedium verwendet werden. Hierzu erweist es sich als zweckmässig, das nach Entspannen des Reaktors in der Salzsäure verbliebene Chlor nicht zu entfernen, sondern gleich die chlorgesättigte Salzsäure weiterzuverwenden. Das Verfahren kann diskontinuierlich aber auch kontinuierlich durchgeführt werden.

Das erfindungsgemässe Verfahren hat gegenüber den eingangs geschilderten Verfahren betreffend die Chlorierung von Hydrochinon in Salzsäure die folgenden Vorteile:

- die Reaktion kann bei weitgehend konstantem Druck durchgeführt werden;
- die Konzentration der als Reaktionsmedium verwendeten Salzsäure bleibt während der Reaktion ebenfalls in etwa konstant;
- die Reaktion und damit die Wärmeentwicklung lässt sich über die Dosiergeschwindigkeit der Ausgangsmaterialien und Chlor leicht steuern;
- es kommt zu keiner Akkumulation an Chinon oder seiner Chlorderivate, so dass nicht die Gefahr einer Ueberhitzung besteht;
- hohe Raum-Zeit-Ausbeuten durch semi-kontinuierliche Reaktionsführung sind möglich, danach wird ein Teil der heissen Reaktionsmasse periodisch aus dem Reaktor abgezogen, die verbleibende Restmenge dient ohne aufzuheizen als Vorlage für den nachfolgenden Ansatz.

Die folgenden Beispiele dienen der Veranschaulichung der Erfindung; Teile bedeuten Gewichtsteile und Prozente Gewichtsprozente.

Beispiel 1: In einem emaillierten Autoklaven werden 1400 Teile 37%ige Salzsäure vorgelegt und auf eine Temperatur von 120°C erhitzt, dabei kommt es zu einem Druckanstieg, der maximal dem Partialdampfdruck der Salzsäure bei dieser Temperatur entspricht (ca. 5 bis 8 bar). Dann wird durch Einleiten von ca. 10 Teilen Chlorgas die Salzsäure mit Chlor gesättigt, wobei der Druck weiter steigt und zwar auf ca. 10 bar. Anschliessend dosiert man während 2 Stunden in die chlorgesättigte Salzsäure gleichzeitig 260 Teile Chlor und 291 Teile einer 25%igen wässrigen Hydrochinon Suspension. Der Reaktorinhalt wird dabei gut gerührt und die Temperatur bei 120°C gehalten. Während der Reaktion ist der Druck weitgehend konstant bei 10 ± 1 bar. Nach beendeter Dosierung lässt man noch 30 Minuten nachreagieren und kühlt dann die Reaktionsmasse auf Raumtemperatur ab. Anschliessend wird das Produkt abgenutscht, gewaschen und getrocknet. Man erhält 157,5 Teile Chloranil, das entspricht einer Ausbeute von 97 %. Das Produkt ist von hoher Reinheit und kann direkt in der Farbstoffsynthese verwendet werden. Im Hochdruck-Flüssigkeits-Chromatogramm hat die Verbindung die gleiche Retentionszeit, wie eine authentische Probe.

Die Mutterlauge besteht aus 37 % Salzsäure und kann ohne weitere Reinigung direkt für einen nachfolgenden Ansatz verwendet werden.

Beispiel 2 (Batch-Verfahren) zum vergleich: 66 Teile Hydrochinon werden in 1000 Teilen Salzsäure 37 % in einem säurebeständigen Autoklaven vorgelegt. Dazu drückt man 222 Teile Chlorgas (Theorie + 4,0 %) und heizt innert 1/2 - 2 Stunden auf 120 °C auf, dabei steigt der Druck gegen 30 bar an. Nach einer Reaktionszeit von 1 bis 4 Stunden bei 120 °C ist die Reaktion beendet. Nach dem Abkühlen auf 25 °C (Ueberdruck ca. 4 bar) wird der Autoklav vorsichtig mit Stickstoff entspannt. Das hellgelbe Produkt wird abgesaugt, mit wenig Wasser gewaschen und im Trockenschrank getrocknet (40 - 50 °C).

Es werden 146 Teile Chloranil erhalten. Das Produkt hat laut Analyse einen Reingehalt von 98 - 99 %. Dies entspricht einer Ausbeute von 97 - 98 % d. Th.

Beispiel 3: In einem emaillierten Autoklaven werden 1400 Teile 37%ige Salzsäure vorgelegt und auf eine Temperatur von 90 °C erhitzt, dabei steigt der Druck auf 3 bar. Dann wird durch Einleiten von ca. 10 Teilen Chlorgas die Salzsäure mit Chlor gesättigt, wobei der Druck weiter steigt und zwar auf ca. 4,0 bar. Anschliessend dosiert man während 8 Stunden in die chlorgesättigte Salzsäure gleichzeitig 290 Teile Chlor und 362 Teile einer 22,8%igen wässrigen Hydrochinon-Lösung von 70 °C. Der Reaktorinhalt wird dabei gut gerührt und die Temperatur bei 90 °C gehalten. Während der Reaktion ist der Druck weitgehend konstant bei 4 ±0,1 bar. Nach beendeter Dosierung lässt man noch 30 Minuten nachreagieren und kühlt dann die Reaktionsmasse auf Raumtemperatur ab. Anschliessend wird das Produkt abgenutscht, gewaschen und getrocknet. Man erhält 179 Teile Chloranil, das entspricht einer Ausbeute von 97 %. Das Produkt ist von hoher Reinheit und kann direkt in der Farbstoffsynthese verwendet werden.

## Patentansprüche

1. Verfahren zur Herstellung von Chloranil durch Chlorieren von Chinon, Hydrochinon oder deren Chlorderivaten in Salzsäure, dadurch gekennzeichnet, dass man die Salzsäure vorlegt und unter Druck Chinon, Hydrochinon oder deren Chlorderivate und Chlor in getrennten Strömen gleichzeitig zudosiert.

2. Verfahren gemäss Anspruch 1, dadurch gekennzeichnet, dass man Hydrochinon oder dessen Chlorderivate chloriert.

3. Verfahren gemäss Anspruch 1, dadurch gekennzeichnet, dass man Hydrochinon oder Chinon und Chlor in einem solchen Verhältnis zudosiert, dass auf 1 Mol Hydrochinon oder Chinon 4 bis 6 Mol Chlor kommen.

4. Verfahren gemäss Anspruch 1, dadurch gekennzeichnet, dass man die Reaktion bei einer Temperatur von 60 bis 150 °C durchführt.

5. Verfahren gemäss Anspruch 4, dadurch gekennzeichnet, dass man die Reaktion bei einer Temperatur von 80 bis 150 °C durchführt.

6. Verfahren gemäss Anspruch 1, dadurch gekennzeichnet, dass man die Reaktion bei einem Druck von 3 bis 40 bar durchführt.

7. Verfahren gemäss Anspruch 6, dadurch gekennzeichnet, dass man die Reaktion bei einem Druck von 5 bis 40 bar durchführt.

8. Verfahren gemäss Anspruch 6, dadurch gekennzeichnet, dass man die Reaktion bei einem Druck von 3 bis 12 bar durchführt.

## Claims

1. A process for the preparation of chloranil by chlorinating quinone, hydroquinone or a chlorinated derivative thereof in hydrochloric acid, which process comprises charging the reactor with hydrochloric acid and, under pressure, simultaneously adding quinone, hydroquinone or a chlorinated derivative thereof and chlorine in separate streams.

2. A process according to claim 1, which comprises chlorinating hydroquinone or a chlorinated derivative thereof.

3. A process according to claim 1, which comprises adding hydroquinone or quinone and chlorine in a ratio such that 4 to 6 moles of chlorine are added per 1 mole of hydroquinone or quinone.

4. A process according to claim 1, which comprises carrying out the reaction in the temperature range from 60 to 150 °C.

5. A process according to claim 4, which comprises carrying out the reaction in the temperature range from 80 to 150 °C.

6. A process according to claim 1, which comprises carrying out the reaction under a pressure of 3 to 40 bar.

7. A process according to claim 6, which comprises carrying out the reaction under a pressure of 5 to 40 bar.

8. A process according to claim 6, which comprises carrying out the reaction under a pressure of 3 to 12 bar.

**Revendications**

1. Procédé pour la préparation du chloranile par chloration de la quinone, de l'hydroquinone ou de leurs dérivés chlorés, dans de l'acide chlorhydrique, caractérisé en ce que l'on dispose au préalable l'acide chlorhydrique et on introduit simultanément de façon réglée, sous pression, en courants séparés, de la quinone, de d'hydroquinone ou leurs dérivés chlorés, et du chlore.

2. Procédé selon la revendication 1, caractérisé en ce que l'on effectue la chloration de d'hydroquinone ou de ses dérivés chlorés.

3. Procédé selon la revendication 1, caractérisé en ce que l'on introduit de façon réglée de d'hydroquinone ou de la quinone et du chlore en un rapport tel que de 4 à 6 modes de chlore arrivent pour 1 mode d'hydroquinone ou de quinone.

4. Procédé selon la revendication 1, caractérisé en ce que la réaction est effectuée à une température de 60 à 150° C.

5. Procédé selon la revendication 4, caractérisé en ce que la réaction est effectuée à une température de 80 à 150° C.

6. Procédé selon la revendication 1, caractérisé en ce que la réaction est effectuée sous une pression de 3 à 40 bars.

7. Procédé selon la revendication 6, caractérisé en ce que la réaction est effectuée sous une pression de 5 à 40 bars.

8. Procédé selon la revendication 6, caractérisé en ce que la réaction est effectuée sous une pression de 3 à 12 bars.